Europäisches Patentamt

European Patent Office

Office européen des brevets

(19)

(11) Numéro de publication: **0 185 597**
**A1**

## (12) DEMANDE DE BREVET EUROPEEN

(21) Numéro de dépôt: **85420106.8**

(22) Date de dépôt: **11.06.85**

(51) Int. Cl.⁴: **A 61 K 6/06**
**A 61 K 6/02**

(30) Priorité: **22.10.84 FR 8416464**

(43) Date de publication de la demande:
**25.06.86 Bulletin 86/26**

(84) Etats contractants désignés:
**BE CH DE GB IT LI NL**

(71) Demandeur: **DURAND GIRARD S.A.**
**22 rue Juiverie**
**F-69005 Lyon(FR)**

(72) Inventeur: **Revel, Francois**
**11 Boulevard des Brotteaux**
**F-69006 Lyon(FR)**

(74) Mandataire: **Caradot, Patrice**
**MAISONNIER, CORNILLON, CARADOT 29 rue de la**
**République B.P. 247**
**F-42006 Saint Etienne Cédex(FR)**

(54) **Produit céramique pour prothèse dentaire.**

(57) L'invention concerne un produit céramique, le procédé pour sa mise en oeuvre à la base d'une prothèse et la prothèse ainsi obtenue. Le produit céramique contient de 90 à 95% de poudre céramique et de 10 à 5% de cire. Ce produit est appliqué sur la base de la prothèse de façon à recouvrir la chape de métal et chauffer à 960°C environ. La Prothèse ainsi obtenue n'a pas de partie métallique apparente au contact de la gencive.
Application: finition ou réparation de prothèse céramique.

Fig : 3

EP 0 185 597 A1

1

PRODUIT CERAMIQUE DE FINITION POUR PROTHESE DENTAIRE

PROCEDE POUR SA MISE EN OEUVRE ET PROTHESE AINSI OBTENUE

L'invention concerne un produit céramique destiné à la finition des prothèses dentaires, le procédé nécessaire à sa mise en oeuvre et la prothèse obtenue par ces moyens.

Afin d'obtenir des prothèses dentaires ne nécessitant pas de métal visible dans la bouche, on utilise des produits céramiques permettant d'obtenir un aspect et une couleur identique à ceux des dents saines du patient. Pour obtenir ce résultat, le chirurgien dentiste taille la dent existante et prend l'empreinte de la taille ainsi réalisée, puis le prothésiste réalise une chape de métal à la forme de cette taille, laquelle chape est recouverte d'une prothèse en céramique. Cette méthode présente l'inconvénient suivant : lorsque la gencive présente un léger retrait, le bord de la chape de métal apparaît, ce qui nuit à l'esthétique de la denture ainsi corrigée. En effet, la gencive a tendance à se retirer lors d'un surcontour ou d'une surépaisseur du au métal. De plus, les reflets du métal donnent un aspect grisâtre à celle-ci.

Pour pallier à cet inconvénient, on a cherché des méthodes permettant de construire ou réparer le joint lors du retrait de la gencive. Un procédé connu consiste à entourer le bas de la taille d'une feuille d'alliage dentaire, par exemple à base d'or sur laquelle on vient déposer au pinceau un joint à base de poudre de céramique. Du fait de l'hétérogénéïté du produit, ce joint est cassant. Sa réalisation après coup est par ailleurs extrêmement onéreuse et peu fiable.

Afin d'éviter ces divers inconvénients, le procédé selon l'invention consiste à réaliser une chape de métal ne venant pas recouvrir le congé de la taille. La prothèse céramique est interrompue à hauteur de la chape ainsi réalisée. On dépose ensuite sur le congé de la taille une couche de vernis, sur laquelle on dépose pour finir à la base de la prothèse un produit céramique formé de la façon suivante :

. 90 % à 95 % de céramique pour prothèse dentaire (poudre de gypse, silice, carolin, quartz etc... de type connu...)

. 5 à 10 % de cire (cire d'abeille, paraffine etc...)

En dessous de 5 % de cire, le mélange ne se réalise pas. Au-délà de 10 %, il y a trop de cire et des vides se produisent lors du séchage du produit mélangé ainsi réalisé.

Le mélange est réalisé à chaud, au moyen par exemple d'une spatule chauffante, la cuisson étant réalisée autour de 960°C.

Le produit ainsi réalisé ne présente pas de retrait lors de son refroidissement et le risque de cassure est évité principalement lors de la désinsertion de la prothèse obtenue. Le joint ainsi réalisé recouvre totalement le congé de la taille. Ainsi, même en cas de léger retrait de la gencive, le métal n'apparaît pas.

Par ailleurs, ce risque de retrait est notoirement diminué par une taille moins sous-gingivale, une prise d'empreinte moins traumatisante et aucun surcontour de métal risque de mettre la gencive en compression.

Ce produit et ce procédé peuvent être utilisés pour la réalisation de prothèses unitaires ou de bridges.

On comprend également que le produit et le procédé selon l'invention peuvent s'appliquer de façon simple à la réparation de prothèses en place.

Les dessins annexés, à titre d'exemple non limitatif, permettront de mieux comprendre les caractéristiques de l'invention.

La figure 1 est une vue d'une prothèse selon le procédé connu.

La figure 2 est une vue d'une prothèse réparée selon une méthode connue.

La figure 3 est une vue d'une prothèse réalisée selon le procédé correspondant à l'invention.

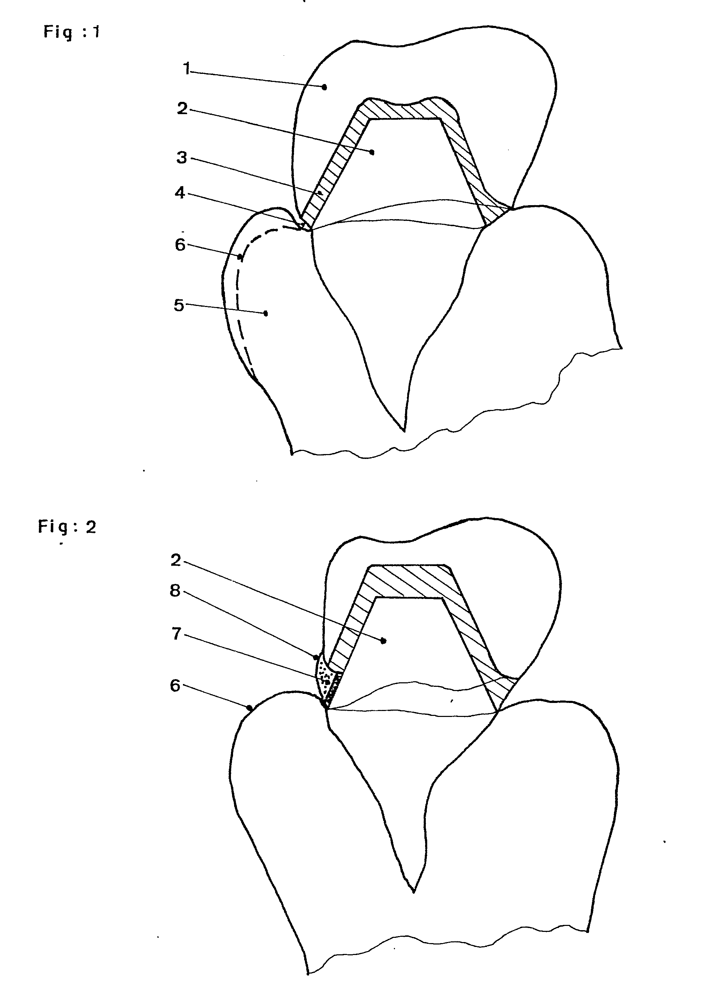

On a représenté sur la figure 1 une vue en coupe d'une prothèse céramique (1) réalisée selon un procédé connu. La taille (2) est recouverte d'une chape (3) métallique sur laquelle est fixée la prothèse céramique (1). Celle-ci vient recouvrir cette chape (3), de telle sorte que la section (4) de cette chape reste visible et vient au contact de la gencive (5). En cas de retrait de la gencive (5),par exemple selon le profil (6), la section (4) de la chape (3) devient visible.

On a représenté sur la figure 2 une vue en coupe d'une prothèse céramique (1) sur laquelle on a procédé à une "réparation" après le retrait de la gencive (6). Une feuille d'alliage dentaire or ou platine (7) est déposée sur la base de la taille (2) puis recouverte de poudre de céramique (8) de type

connu.

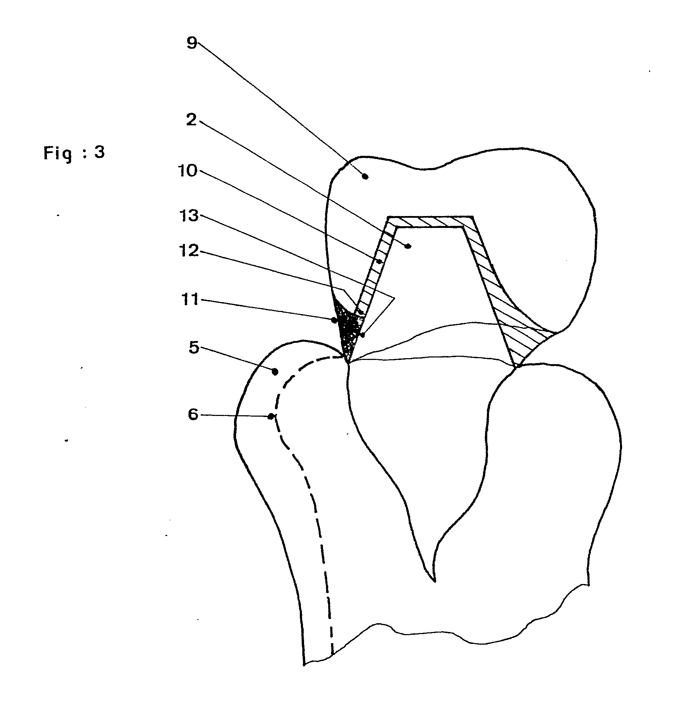

On a représenté sur la figure 3 une prothèse céramique (9), réalisée au moyen du procédé et du produit selon l'invention. La chape métallique (10) est interrompue avant la base de la taille (2). La prothèse (9) est alors complétée par un bourrelet (11) formé du produit selon l'invention mis en oeuvre par le procédé précédemment décrit. Ce bourrelet (11) recouvre la section (12) de la chape (10) et l'extrémité de la prothèse (9) de telle façon qu'aucune solution de continuité n'intervienne. On comprend que reposant sur le congé cervical (13) de la taille (2) même en retrait de la gencive (5) selon le profil (6), l'extrémité de la chape (10) n'est plus visible. Par ailleurs, ce retrait est beaucoup moins fréquent du fait que la gencive (5) n'est pas mise en compression par un sertissage métallique.

4

**0185597**

REVENDICATIONS

1 - Produit céramique pour la finition de prothèses dentaires céramiques destiné à s'interposer entre la gencive et la prothèse, caractérisé en ce qu'il est formé d'un mélange de poudre de céramique entre 90 et 95 % et de cire entre 5 et 10 % mélangé à chaud.

2 - Procédé pour la mise en oeuvre du produit céramique selon la revendication 1, caractérisé en ce que le produit est déposé selon un bourrelet (11) sur une couche de vernis appliqué sur la feuille (2) et cuit à une température comprise entre 940° C et 980° C.

3 - Procédé pour la mise en oeuvre du produit céramique selon la revendication 2, caractérisé en ce que le produit formant le bourrelet (11) est appliqué sur le congé (13) de la taille (2).

4 - Prothèse céramique obtenue par la mise en oeuvre du produit selon la revendication (2) caractérisé en ce que la section (12) de la chape métallique (10) de la prothèse (9) est intégralement recouverte par le bourrelet (11) formé par le produit céramique selon l'invention.

0185597

Fig : 1

Fig : 2

Fig : 3

Office européen
des brevets

**RAPPORT DE RECHERCHE EUROPEENNE**

0185597

Numéro de la demande

EP 85 42 0106

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl.4) |
|---|---|---|---|
| X | US-A-1 621 793 (P.W. KRUGER) * Page 1, lignes 1-4; page 1, lignes 52-65; page 2, lignes 41-49; revendications 1 et 2 * | 1 | A 61 K 6/06 A 61 K 6/02 |
| X | DE-C- 447 088 (RICHTER & HOFMANN HARRARD) * En entier * | 1 | |
| X | DE-C- 558 593 (O. SIMON) * Page 1, lignes 10-25; page 1, lignes 50-59; page 2, lignes 65-75; revendications 1 et 2 * | 1,2 | |
| A | CHEMICAL ABSTRACTS, vol. 95, no. 22, novembre 1981, page 350, résumé 192448h, Columbus, Ohio, US; & SU - A - 856 459 (LENINGRAD FIRST MEDICAL INSTITUTE) 23.08.1981 * Résumé * | 1 | DOMAINES TECHNIQUES RECHERCHES (Int. Cl.4) A 61 K |
| A | DE-C- 292 406 (Dr. G. GASPARI) * En entier * | 1-4 | |

Le présent rapport de recherche a été établi pour toutes les revendications

| Lieu de la recherche | Date d'achèvement de la recherche | Examinateur |
|---|---|---|
| LA HAYE | 07-02-1986 | BERTE M.J. |